# EUROPEAN PATENT SPECIFICATION

(11) **EP 1 720 528 B1**
(45) Date of publication and mention of the grant of the patent: **04.07.2012**
(21) Application number: 05728271.7
(22) Date of filing: 11.02.2005
(51) Int. Cl.: A61K 9/51, A61L 31/16, A61L 31/12, A61L 29/16, A61L 29/12

(54) **LAYERED SILICATE NANOPARTICLE-POLYMER COMPOSITE MEDICAL ARTICLES**
MEDIZINISCHE ARTIKEL MIT VERBUNDMATERIAL AUS SCHICHTSILIKAT-NANOPARTIKELN UND POLYMER
ARTICLES MEDICAUX COMPRENANTS UN MATERIAU COMPOSITE DE NANOPARTICLES DE SILICATE STARTIFIE DE POLYMERE

(30) Priority: 12.02.2004 US 777802
(43) Date of publication of application: 15.11.2006
(73) Proprietor: Boston Scientific Limited, Barbados, West Indies (BB)
(72) Inventor: ZHONG, Sheng-Ping (Samuel), Shrewsbury, MA 01545 (US)
(74) Representative: Vossius & Partner
(86) International application number: PCT/US2005/004585
(87) International publication number: WO 2005/079754

(56) References cited:
- EP-A- 1 470 823
- WO-A-03/026532
- WO-A-2004/098574
- US-A1- 2003 143 350
- CYPES S H ET AL: "Organosilicate-polymer drug delivery systems: controlled release and enhanced mechanical properties" JOURNAL OF CONTROLLED RELEASE, ELSEVIER SCIENCE PUBLISHERS B.V. AMSTERDAM, NL, vol. 90, no. 2, 24 June 2003 (2003-06-24), pages 163-169, XP004431309 ISSN: 0168-3659
- ALEXANDRE M ET AL: "Polymer-layered silicate nanocomposites: preparation, properties and uses of a new class of materials" MATERIALS SCIENCE AND ENGINEERING R: REPORTS, ELSEVIER SEQUOIA S.A., LAUSANNE, CH, vol. 28, no. 1-2, June 2000 (2000-06), pages 1-63, XP004205143 ISSN: 0927-796X

## Description

### FIELD OF THE INVENTION

The present invention relates to medical articles which are useful for the controlled delivery of therapeutic agents.

### BACKGROUND OF THE INVENTION

Medical articles are frequently used for the delivery of therapeutic agents. For example, an implantable or insertable medical device, such as a stent or a catheter, may be provided with a polymer matrix coating layer that contains a therapeutic agent. Once the medical device is placed at a desired location within a patient, the therapeutic agent is released from the polymer matrix and into the patient, thereby achieving a desired therapeutic outcome.

It is a daunting challenge, however, to qualify a polymer for use in such a polymer matrix. For example, the polymer has to meet a long list of requirements related to its mechanical, chemical, biological characteristics. Moreover, the polymer should be readily formulated with the therapeutic agent. In some cases, the polymer and therapeutic agent are both hydrophobic (or they are both hydrophilic) and they share the same solvent, in which case blending the drug in the polymer matrix is relatively straightforward. However, where the drug is hydrophilic and the polymer is hydrophobic, or vice versa, attempts to blend the drug into the polymer commonly result in unstable formulations with accompanying phase separation.

Layered silicate materials such as smectite clays are well known. The atoms within single layers of these materials are tightly bound together, but the forces between adjacent layers are relatively weak. A typical clay particle can consist of from two to hundreds of such layers or more. The layers have inorganic cations such as calcium, magnesium, potassium, sodium, and hydrogen on their external and inter-layer surfaces. These cations balance the net negative charges that exist within the layers. As a result, two adjacent negatively charged layers are held together by the presence of the cations that are situated between the layers. This bonding, although strong enough to keep the layers together, is much weaker than the covalent bonds that exist between the atoms within the layers themselves. This weaker bonding between layers, plus the strong attraction of the interlayer cations for water and other polar molecules, allows these molecules to enter into the interlayer space.

One consequence of the above characteristics is that the endogenous inorganic interlayer cations of layered silicate materials can be displaced by other inorganic or organic cations that are present within a surrounding liquid. Thus, if layered silicate particles are suspended in a liquid that contains cations, for example, a molecular species having a cationic charge and a hydrophobic domain, there is commonly an exchange of the endogenous inorganic interlayer cations of the silicate for cations in the surrounding liquid. (Hence, these cations are sometimes referred to as exchangeable cations.) For instance, many layered silicates including synthetic or naturally occurring smectites, are relatively hydrophilic, and the addition of organic cations (such as cationic quaternary ammonium compounds having hydrophobic domains) to replace the inorganic cations occupying the exchange sites has been shown to render the clay more lipophilic.

### SUMMARY OF THE INVENTION

The present invention is directed to novel release regions for controlling the release of therapeutic agents from medical articles. Upon placement of such a medical article at a position on or within a patient, the release region regulates the rate of release of the therapeutic agent from the medical article to the patient.

According to one aspect of the present invention, a medical article as disclosed in claim 1 (for instance, a drug delivery patch or an implantable or insertable medical device, among others) is provided, which comprises a release region. The release region, in turn, comprises (a) polymeric carrier comprising a polymer (for instance, a hydrophobic, hydrophilic or amphiphilic polymer) and (b) drug loaded nanoparticles, which are dispersed within the polymeric carrier. The drug loaded nanoparticles, in turn, comprise a layered silicate material (for instance, a synthetic or naturally occurring smectite material) and a therapeutic agent (for instance, a hydrophobic, hydrophilic or amphiphilic therapeutic agent).

Another aspect of the present invention is directed to methods of producing such medical articles.

Yet another aspect of the present invention is directed to methods of releasing a therapeutic agent by contacting (e.g., adhering, implanting, inserting, and so forth) the above medical articles with patients.

An advantage of the present invention is that medical articles can be provided, which regulate the release of therapeutic agent from a medical article to a patient.

Another advantage of the present invention is that it allows hydrophobic therapeutic agents to be incorporated into hydrophilic carrier regions, and vice versa.

As an additional benefit, the nanoparticles can improve the mechanical properties of medical articles.

These and other aspects, embodiments and advantages of the present invention will become immediately apparent to those of ordinary skill in the art upon review of the Detailed Description and Claims to follow.

### BRIEF DESCRIPTION OF THE DRAWINGS

Fig. 1 is a schematic illustration of a stent, in accordance with an embodiment of the invention.

Fig. 2 is a schematic cross-sectional illustration of a structural element of a stent like that of Fig. 1, in accordance with an embodiment of the invention.

Fig. 3 is a schematic cross-sectional illustration of a structural element of a stent like that of Fig. 1, in accordance with another embodiment of the invention.

### DETAILED DESCRIPTION OF THE INVENTION

According to an aspect of the present invention, a medical article is provided, which comprises a novel release region. The release region comprises (a) a polymeric carrier, which includes one or more polymers, and (b) drug loaded nanoparticles, which are dispersed within the polymeric carrier. The drug loaded nanoparticles comprise a layered silicate material and a therapeutic agent.

In some embodiments of the invention, the release region constitutes only a portion of the medical article, for example, where the release region constitutes a component of the medical article, or where it is disposed as a layer over all or a portion of a medical article substrate. In other embodiments, the release region constitutes the entirety of the medical article.

As used herein a "layer" of a given material is a region of that material whose thickness is small compared to both its length and width (e.g., the length and width dimensions may both be at least 5, 10, 20, 50, 100 or more times the thickness dimension in some embodiments). As used herein a layer need not be planar, for example, taking on the contours of an underlying substrate. Layers can be discontinuous (e.g., patterned). Terms such as "film," "layer" and "coating" may be used interchangeably herein.

Medical articles of the present invention include any medical article for which controlled release of a therapeutic agent is desired. Examples of medical articles include patches for delivery of therapeutic agent to intact skin, broken skin (including wounds), and surgical sites.

Examples of medical articles also include implantable or insertable medical devices, for instance, catheters (e.g., renal or vascular catheters such as balloon catheters), guide wires, balloons, filters (e.g., vena cava filters), stents (including coronary vascular stents, cerebral, urethral, ureteral, biliary, tracheal, gastrointestinal and esophageal stents), stent grafts, cerebral aneurysm filler coils (including Guglilmi detachable coils and metal coils), vascular grafts, myocardial plugs, patches, pacemakers and pacemaker leads, electrodes, heart valves, circulation pumps, biopsy devices, and any other coated substrate (which can comprise, for example, glass, metal, polymer, ceramic and combinations thereof) that is implanted or inserted into the body.

The medical articles of the present invention include medical articles that are used for either systemic treatment or for the localized treatment of any mammalian tissue or organ. Examples include tumors; organs including the heart, coronary and peripheral vascular system (referred to overall as "the vasculature"), lungs, trachea, esophagus, brain, liver, kidney, bladder, urethra and ureters, eye, intestines, stomach, pancreas, ovary, and prostate; skeletal muscle; smooth muscle; breast; dermal tissue; cartilage; and bone.

As used herein, "treatment" refers to the prevention of a disease or condition, the reduction or elimination of symptoms associated with a disease or condition, or the substantial or complete elimination a disease or condition. Preferred subjects are mammalian subjects and more preferably human subjects.

Medical articles having sustained releases profiles are beneficial in many cases. By "sustained release profile" is meant a release profile in which less than 25% of the total release from the medical article that occurs over the entire course of administration occurs after 1 day (or in some embodiments, after 2, 4, 8, 16, 32, 64, 128 or even more days) of administration. Conversely, this means that more than 75% of the total release from the medical device will occur after the device has been administered for the same period.

The release characteristics that are ultimately of interest are, of course, the release characteristics within the subject, for example, within a mammalian subject. However, it is well known in the art to test the release characteristics within an experimental system that gives an indication of the actual release characteristics within the subject. For example, an aqueous buffer system, such as Tris buffer or phosphate buffered saline, is commonly used for testing release of therapeutic agents from vascular medical devices.

Specific examples of medical articles for use in conjunction with the present invention include vascular stents that are used to deliver therapeutic agent into the vasculature for the treatment of restenosis. In these embodiments, a release layer in accordance with the present invention is typically provided over all or a portion of a stent substrate.

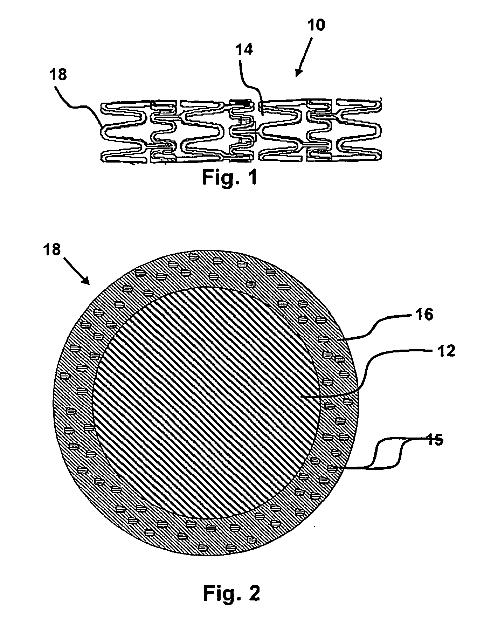

In this connection, Fig. 1 illustrates a vascular stent 10, in accordance with an embodiment of the present invention. Stent 10 can be, for example, a coronary stent, sized to fit in the blood vessel of a patient, which is formed from a plurality of structural elements18. The construction of stent 10 permits the stent 10 to be introduced into the vascular system in a collapsed configuration, minimizing the diameter of the stent 10. Stent 10 can then expand to an expanded position at the desired location within the blood vessel of the patient. The structural elements 18 of stent 10 form a frame, such as tubular shape, permitting the stent 10 to self-expand or to expand to the desired shape after an expansive force is applied, for example, by the expansion of a balloon within the stent. The structural elements 18 of stent 10 form windows 14 such that the stent 10 does not have a continuous outer shell. Windows 14 are generally present in most stent configurations, although the specific details of the shape of structural elements 18 and the construction of stent 10 can vary.

In some embodiments, a release layer in accordance with the present invention is applied on the surface of a stent. For example, Fig. 2 is a schematic cross-sectional view of a structural element 18 of a stent like that of Fig. 1, in accordance with an embodiment of the invention. In Fig. 2, the release layer 16 includes a plurality of drug loaded nanoparticles 15, dispersed within a polymeric carrier. As indicated above, the drug loaded nanoparticles 15 comprise a therapeutic agent in association with particles of a layered silicate material. The release layer 16 is directly adjacent the underlying structural member 12, which acts as a substrate for the release layer 16.

Layered silicate particles (sometimes referred to as "phyllosilicates") for the practice of the present invention can be selected from natural or synthetic layered silicate particles and typically have a maximum cross-sectional length (for instance, the diameter in the case of a spherical particle or the width in the case of a plate-shaped particle) between 1 and 1000 nanometers, more typically between 30 to 500nm. The spacing between the adjacent layers within the silicate particles is typically in the range of 5-20A.

Layered silicate particles for the practice of the present invention can be selected from natural and synthetic versions of following: (a) allophane; (b) apophyllite; (c) bannisterite; (d) carletonite; (e) cavansite; (f) chrysocolla; (g) members of the clay group, including: (i) members of the chlorite group such as baileychlore, chamosite, the mineral chlorite, clinochlore, cookeite, nimite, pennantite, penninite, sudoite, (ii) glauconite, (iii) illite, (iv) kaolinite, (v) montmorillonite, (vi) palygorskite, (vii) pyrophyllite, (viii) sauconite, (ix) talc, and (x)vermiculite; (h) delhayelite; (i) elpidite; (j) fedorite; (k) franklinfurnaceite; (l) franklinphilite; (m) gonyerite; (n) gyrolite; (o) leucosphenite; (p) members of the mica group, including (i) biotite, (ii) lepidolite, (iii) muscovite, (iv) paragonite, (v) phlogopite, and (vi) zinnwaldite; (q) minehillite; (r) nordite; (s) pentagonite; (t) petalite; (u) prehnite; (v) rhodesite; (w) sanbomite; (x) members of the serpentine group, including (i) antigorite, (ii) clinochrysotile, (iii) lizardite, (iv) orthochrysotile and (v) serpentine; (y) wickenburgite; (z) zeophyllite; and mixtures thereof.

Additional layered silicate materials for the practice of the present invention, not necessarily exclusive of those above, can be selected from natural and synthetic versions of following: aliettite, swinefordite, yakhontovite, volkonskoite, stevensite, hectorite, magadiite, kenyaite, ledikite, laponite, saponite, sauconite, montmorillonite, bentonite, nontronite, beidellite, hectorite, other smectite group clays, and mixtures thereof.

Depending on the nature of the therapeutic agent and the nature of the layered silicate particles, the therapeutic agent may be maintained in association with the layered silicate particles by any of a number of mechanisms including, for example, hydrogen bonding, Van der Waals bonding, bonding through hydrophilic/hydrophobic interactions, ionic bonding, and so forth. By associating the therapeutic agent with the silicate particles, each silicate particle becomes a miniature depot for the therapeutic agent. Preferably, the therapeutic agent is associated with the silicate particle in a way such that it occupies the spaces between adjacent layers of the silicate particle.

In some embodiments of the invention, for example, where the therapeutic agent and the layered silicate are both of similar hydrophilicity (or are both of similar hydrophobicity), the therapeutic agent can spontaneously associate with the layers of the silicate particles. For instance, as noted above, the interlayer cations commonly exhibit a strong attraction for polar molecules, and thus for therapeutic agents having polar characteristics.

In other embodiments, for example, where the therapeutic agent is relatively hydrophobic and the layered silicate particles are relatively hydrophilic, the layered silicate can be rendered more hydrophobic by exchanging endogenous inorganic cations found within the silicate particles with one or more species having a positive charge and having a hydrophobic domain as is known in the layered silicate art. Examples of such species include alkylammonium ions, for instance, tertiary and quaternary alkylammonium ions, such as trimethyl ammonium ions and hexadecyltrimethylammonium (HDTMA) ions. By replacing the inorganic cations of the layered silicate with these intercalated species, the layered silicate is rendered more hydrophobic, thereby enhancing the association between the relatively hydrophobic therapeutic agent and the layered silicate. In these embodiments, the therapeutic agent is beneficially introduced concurrently with or subsequent to the introduction of the exchangeable cation.

Additional species other than alkyammonium ions for intercalation between the silicate nanoparticle layers (and hence for varying the interlayer environment) are known, and include those described in U.S: Patent Nos. 6,057,396 and 6,083,559. These species include the following: (a) organic compounds comprising an alkyl radical of at least six carbons and a polar functionality, for example, alcohols and polyalcohols, carbonyl compounds (including carboxylic acids, polycarboxylic acids, and salts thereof), aldehydes, ketones, amines, amides, ethers, esters, lactams, lactones, anhydrides, alkyl nitriles, n-alkyl halides and pyridines, and (b) organic compounds having hydroxyl, polyhydroxyl, and/or aromatic functionality, for example, aliphatic alcohols, aromatic alcohols, aryl substituted aliphatic alcohols, alkyl substituted aromatic alcohols, and polyhydric alcohols. As with the alkyammonium ions above, the therapeutic agent is beneficially introduced concurrently with or subsequent to the introduction of the additional species.

In still other embodiments of the invention, the layered silicate particles are surface modified to carry various charges to bind certain drugs. For instance, in some embodiments, the silicate particles are modified to carry cationic charges or anionic charges. Moreover, in some embodiments, the silicate particles are modified to carry certain functional groups. For instance, a number of grafting techniques are known in the silicate art for establishing various functional groups on the surfaces of layered silicate particles, including hydrophobic and ionic functional groups.

Once formed, the drug loaded nanoparticles of the present invention have great flexibility with respect to (a) the range of polymeric carriers into which they can be incorporated, and (b) the techniques by which they can be formulated into the polymeric carriers.

As a result, the polymers for use in the polymeric carriers of the invention may be homopolymers or copolymers (including alternating, random and block copolymers), they may be cyclic, linear or branched (e.g., polymers have star, comb or dendritic architecture), they may be natural or synthetic, they may be thermoplastic or thermosetting, and they may be hydrophobic, hydrophilic or amphiphilic.

Polymers for use in the polymeric carriers may be selected, for example, from the following: polycarboxylic acid polymers and copolymers including polyacrylic acids; acetal polymers and copolymers; acrylate and methacrylate polymers and copolymers (e.g., n-butyl methacrylate); cellulosic polymers and copolymers, including cellulose acetates, cellulose nitrates, cellulose propionates, cellulose acetate butyrates, cellophanes, rayons, rayon triacetates, and cellulose ethers such as carboxymethyl celluloses and hydoxyalkyl celluloses; polyoxymethylene polymers and copolymers; polyimide polymers and copolymers such as polyether block imides, polyamidimides, polyesterimides, and polyetherimides; polysulfone polymers and copolymers including polyarylsulfones and polyethersulfones; polyamide polymers and copolymers including nylon 6,6, nylon 12, polycaprolactams and polyacrylamides; resins including alkyd resins, phenolic resins, urea resins, melamine resins, epoxy resins, allyl resins and epoxide resins; polycarbonates; polyacrylonitriles; polyvinylpyrrolidones (cross-linked and otherwise); polymers and copolymers of vinyl monomers including polyvinyl alcohols, polyvinyl halides such as polyvinyl chlorides, ethylene-vinylacetate copolymers (EVA), polyvinylidene chlorides, polyvinyl ethers such as polyvinyl methyl ethers, polystyrenes, styrene-maleic anhydride copolymers, styrene-butadiene copolymers, styrene-ethylenebutylene copolymers (e.g., a polystyrene-polyethylene/butylene-polystyrene (SEBS) copolymer, available as Kraton® G series polymers), styrene-isoprene copolymers (e.g., polystyrene-polyisoprene-polystyrene), acrylonitrile-styrene copolymers, acrylonitrile-butadiene-styrene copolymers, styrene-butadiene copolymers and styrene-isobutylene copolymers (e.g., polyisobutylene-polystyrene block copolymers such as SIBS), polyvinyl ketones, polyvinylcarbazoles, and polyvinyl esters such as polyvinyl acetates; polybenzimidazoles; polyalkyl oxide polymers and copolymers including polyethylene oxides (PEO); polyesters including polyethylene terephthalates and aliphatic polyesters such as polymers and copolymers of lactide (which includes lactic acid as well as d-,land meso lactide), epsilon-caprolactone, glycolide (including glycolic acid), hydroxybutyrate, hydroxyvalerate, para-dioxanone, trimethylene carbonate (and its alkyl derivatives), 1,4-dioxepan-2-one, 1,5-dioxepan-2-one, and 6,6-dimethyl-1,4-dioxan-2-one (a copolymer of polylactic acid and polycaprolactone is one specific example); polyether polymers and copolymers including polyarylethers such as polyphenylene ethers, polyether ketones, polyether ether ketones; polyphenylene sulfides; polyolefin polymers and copolymers, including polyalkylenes such as polypropylenes, polyethylenes (low and high density, low and high molecular weight), polybutylenes (such as polybut-1-ene and polyisobutylene), EPDM copolymers (e.g., santoprene), ethylene propylene diene monomer (EPDM) rubbers, poly-4-methyl-pen-1-enes, ethylene-alpha-olefin copolymers, ethylene-methyl methacrylate copolymers and ethylene-vinyl acetate copolymers; fluorinated polymers and copolymers, including polytetrafluoroethylenes (PTFE), poly(tetrafluoroethylene-co-hexafluoropropene) (FEP), modified ethylene-tetrafluoroethylene copolymers (ETFE), and polyvinylidene fluorides (PVDF); silicone polymers and copolymers; polyurethanes; p-xylylene polymers; polyiminocarbonates; copoly(ether-esters) such as polyethylene oxide-polylactic acid copolymers; polyphosphazines; polyalkylene oxalates; polyoxaamides and polyoxaesters (including those containing amines and/or amido groups); polyorthoesters; biopolymers, such as polypeptides, proteins, polysaccharides and fatty acids (and esters thereof), including fibrin, fibrinogen, collagen, elastin, chitosan, gelatin, starch, glycosaminoglycans such as hyaluronic acid; as well as blends and copolymers of the above.

Elastomeric polymers are particularly beneficial in some embodiments. Among the elastomeric polymers are included (a) polyolefin polymers, for example, butyl containing polymers such as polyisobutylene, (b) polyolefin copolymers, for example, polyolefin-polyvinylaromatic copolymers such as polyisobutylene-polystyrene copolymers, poly(butadiene/butylene)-polystyrene copolymers, poly(ethylene/butylene)-polystyrene copolymers, and polybutadiene-polystyrene copolymers; and (c) silicone polymers and copolymers; as well as blends thereof. Specific examples of polyolefin-polyvinylaromatic copolymers include polyolefin-polyvinylaromatic diblock copolymers and polyvinylaromatic-polyolefin-polyvinylaromatic triblock copolymers, such as a polystyrene-poly(ethylene/butylene)-polystyrene (SEBS) triblock copolymer, available as Kraton®, and polystyrene-polyisobutylene-polystyrene (SIBS) triblock copolymers, which are described, for example, in U.S. Patent No. 5,741,331, U.S. Patent No. 4,946,899 and U.S. Patent No. 6,545,097. Additional polyolefin-polyvinylaromatic copolymers are set forth in the prior paragraph.

In some embodiments of the invention the medical article contains a hydrophobic polymer, a hydrophilic polymer, or both a hydrophobic polymer and a hydrophilic polymer.

Examples of hydrophobic polymers from which the polymers used in the present invention can be selected include: olefin polymers and copolymers, such as polyethylene, polypropylene, poly(1-butene), poly(2-butene), poly(1-pentene), poly(2-pentene), poly(3-methyl-1-pentene-), poly(4-methyl- 1-pentene), poly(isoprene), poly(4-methyl-1-pentene), ethylene-propylene copolymers, ethylene-propylene-hexadiene copolymers, ethylene-vinyl acetate copolymers; styrene polymers and copolymers such as poly(styrene), poly(2-methylstyrene), styrene-acrylonitrile copolymers having less than about 20 mole-percent acrylonitrile, styrene-2,2,3,3,-tetrafluoropropyl methacrylate copolymers and olefin-styrene copolymers; halogenated hydrocarbon polymers and copolymers such as poly(chlorotrifluoroethylene), chlorotrifluoroethylene-tetrafluoroethylene copolymers, poly(hexafluoropropylene), poly(tetrafluoroethylene), tetrafluoroethylene, tetrafluoroethylene-ethylene copolymers, poly(trifluoroethylene), poly(vinyl fluoride), poly(vinyl chloride) and poly(vinylidene fluoride); vinyl polymers and copolymers, such as poly(vinyl butyrate), poly(vinyl decanoate), poly(vinyl dodecanoate), poly(vinyl hexadecanoate), poly(vinyl hexanoate), poly(vinyl propionate), poly(vinyl octanoate), poly(heptafluoroisopropoxyethylene), poly(heptafluoroisopropoxypropylene) and poly(methacrylonitrile); polymers and copolymers of acrylic acid esters, such as poly(n-butyl acrylate), poly(ethyl acrylate), poly(1-chlorodifluoromethyl)tetrafluoroethyl acrylate, poly di(chlorofluoromethyl)fluoromethyl acrylate, poly(1,1-dihydroheptafluorobutyl acrylate), poly(1,1-dihydropentafluoroisopropyl acrylate), poly(1,1-dihydropentadecafluorooctyl acrylate), poly(heptafluoroisopropyl acrylate), poly 5-(heptafluoroisopropoxy)pentyl acrylate, poly 11-(heptafluoroisopropoxy)undecyl acrylate, poly 2-(heptafluoropropoxy)ethyl acrylate and poly(nonafluoroisobutyl acrylate); polymers and copolymers of methacrylic acid esters, such as poly(benzyl methacrylate), poly(methyl methacrylate), poly(n-butyl methacrylate), poly(isobutyl methacrylate), poly(t-butyl methacrylate), poly(t-butylaminoethyl methacrylate), poly(dodecyl methacrylate), poly(ethyl methacrylate), poly(2-ethylhexyl methacrylate), poly(n-hexyl methacrylate), poly(phenyl methacrylate), poly(n-propyl methacrylate), poly(octadecyl methacrylate), poly(1,1-dihydropentadecafluorooctyl methacrylate), poly(heptafluoroisopropyl methacrylate), poly(heptadecafluorooctyl methacrylate), poly(1-hydrotetrafluoroethyl methacrylate), poly(1,1-dihydrotetrafluoropropyl methacrylate), poly(1-hydrohexafluoroisopropyl methacrylate), and poly(t-nonafluorobutyl methacrylate); polycarbonates; polyimides; polyetheretherkeones; polyamides; polyvinylaceteates; polysulfones, polyethersulfones; polyesters, such a poly(ethylene terephthalate) and poly(butylene terephthalate); polyurethanes and siloxane-urethane copolymers; and polyorganosiloxanes (silicones).

Examples of hydrophilic polymers from which the polymers used in the present invention can be selected include: polymers and copolymers of acrylic and methacrylic acid, and alkaline metal and ammonium salts thereof; polymers and copolymers of methacrylamide; polymers and copolymers of methacrylonitrile; polymers and copolymers of unsaturated dibasic acids, such as maleic acid and fumaric acid, and half esters of these unsaturated dibasic acids, as well as alkaline metal or ammonium salts of these dibasic adds or half esters; polymers and copolymers of unsaturated sulfonic acids, such as 2-acrylamido-2-methylpropanesulfonic acid and 2-(meth)acryloylethanesulfonic acid, and alkaline metal and ammonium salts thereof; polymers and copolymers of methacrylate esters with hydrophilic groups such as 2-hydroxyethyl methacrylate and 2-hydroxypropylmethacrylate, polymers and copolymers of polyvinyl alcohol, which may contain a plurality of hydrophilic groups such as hydroxyl, amido, carboxyl, amino, ammonium and sulfonyl (--SO₃) groups; polymers and copolymers of polyalkylene glycols and oxides such as polyethylene glycol, polypropylene glycol, polyethylene oxide and polypropylene oxide; polymers and copolymers of vinyl compound having polar pendant groups such as N-vinylpyrrolidone, N-vinyl butyrolactam, N-vinyl caprolactam; polymers and copolymers derived from acrylamide; hydrophilic polyurethanes; polymers and copolymers of hydroxy acrylate; polymers and copolymers of vinylpyrrolidone including vinyl actetate/vinyl pyrrolidone copolymers, starches, polysaccharides including gums and cellulosic polymers such as guar, xanthan and other gums, dextrans, hydroxypropyl cellulose, methyl cellulose, carboxymethyl cellulose, collagen, gelatin, alginate, and hyaluronic acid.

Regardless of their composition, the polymeric carriers of the present invention will typically meet all of the mechanical, chemical and biological requirements of the medical article, and will typically have good adhesion to any underlying substrate.

As noted above, the drug loaded nanoparticles of the present invention also have great flexibility with respect to the techniques by which they can be formulated into the polymeric carriers of the invention.

For example, where the release region is processed using thermoplastic techniques, the nanoparticles can be dispersed in the carrier polymer(s) while heating the polymer(s) to a melt stage. The nanoparticles are dispersed, for example, by applying shear, while at the same time adjusting viscosity so that the particles are remain suspended, and do not settle or aggregate. Hence, depending on the degree of compatibility between the nanoparticles and the polymer(s), the viscosity of the melt can be increased or decreased, for example, by decreasing or increasing temperature of the melt, respectively. Upon cooling, the nanoparticles become entrapped in the polymer(s).

In other embodiments, for example, where the polymeric carrier is processed using solution-based techniques, the carrier polymer(s) is(are) dissolved in one or more solvents to form a solution, and the nanoparticles are dispersed in the resulting solution. The nanoparticles become entrapped in the carrier polymer(s) upon removal of the solvent(s). Depending on the degree of compatibility between the nanoparticles and the polymer(s), the viscosity of the solution can be increased or decreased by decreasing or increasing the amount of solvent(s), respectively. For example, where a hydrophilic layered silicate is dispersed within a hydrophobic polymeric carrier, it may be desirable to increase the viscosity of the polymer solution to ensure that the nanoparticles are maintained in a substantially dispersed state prior to solvent removal.

In some embodiments of the invention, the nanoparticles are loaded with one or more materials in addition to therapeutic agents. For instance, in some embodiments, the nanoparticles are loaded with polymeric materials, along with the therapeutic agent(s). Polymers for such use can be selected, for example, from the polymers listed above. In these embodiments, drug loading and drug release are influenced by the polymer that is co-loaded with the drug.

As with the drug, in some embodiments, the introduction of polymers into the interlayer regions of the nanoparticles is enhanced using species such as the alkylammonium or other intercalation species described above. In this regard, the polymer to be loaded is beneficially introduced concurrently with, or subsequent to, the introduction of the intercalation species. In other embodiments, the polymer is introduced without such intercalation species. For example, (a) intercalation of PEO into Namontmorillonite and Li-montmorillonite by heating has been reported, as has the intercalation of polyvinylpyrrolidone (PVP) between monoionic montmorillonite clay platelets (Na, K, Ca and Mg) by successive washes with absolute ethanol and PVP/ethanol/water solutions, and the intercalation of polyvinyl alcohols containing residual acetyl groups from solution containing the polymer. For further details see, e.g., U.S. Patent Nos. 6,057,396 as well as Richard A. Vaia, et al., "New Polymer Electrolyte Nanocomposites: Melt Intercalation of Poly(Ethylene Oxide) in Mica-Type Silicates", Adv. Materials, Vol. 7, No. 2, pp, 154-156 (1985); Levy, et al., "Interlayer Adsorption of Polyvinylpyrrolidone on Montmorillonite", Journal of Colloid and Interface Science, Vol. 50, No. 3, pp. 442-450 (March 1975); Greenland, "Adsorption of Polyvinyl Alcohols by Montmorillonite", Journal of Colloid Sciences, Vol. 18, pp. 647-664 (1963).

As a specific example, a hydrophilic drug such as halofuginone·HBr, a drug used to treat restenosis, is optionally mixed with a hydrophilic polymer (e.g., dextran, hyaluronic acid or polyethylene oxide), and the drug or the drug/polymer mixture is mutually associated with silicate nanoparticles (for instance, such that they occupy the spaces between adjacent layers of the silicate nanoparticles). If desired, the optional hydrophilic polymer can be crosslinked (a) to prevent the polymer from dissolving (although it will still swell) and/or (b) to regulate drug diffusion rate.

As another specific example, nanoparticles are loaded with a hydrophobic drug such as paclitaxel and, optionally, a hydrophobic polymer, for example, by first rendering the silicate nanoparticles more hydrophobic via cation exchange (or via exposure to another intercalation species as discussed above) prior to exposure to the drug or the drug/polymer mixture.

Subsequently, the drug-loaded nanoparticles are blended, for example, into a melt or a solution containing a carrier polymer.

For example, in some embodiments, the drug-loaded nanoparticles are blended into a melt or a solution containing a hydrophobic carrier polymer (e.g., SIBS or a more radiation stable hydrophobic polymer such as SEBS). So long as the viscosity of the melt or solution is kept sufficiently high (e.g., by keeping the melt temperature low or by limiting the amount of solvent in the solution, e.g., toluene), the drug-and-polymer containing nanoparticles are substantially uniformly suspended in the hydrophobic polymer. Alternatively, suspendablility of the nanoparticles is improved by modifying the surface of the drug and polymer loaded nanoparticles to be more hydrophobic, for example, by grafting hydrophobic species onto the surfaces of the nanoparticles.

In other embodiments, the drug-loaded nanoparticles are blended into a melt or solution (e.g., an aqueous solution) containing a hydrophilic carrier polymer (e.g., collagen, hyaluronic acid, heparin, chrondroitin sulfate or phosphorocholine). So long as the viscosity of the melt or solution is kept sufficiently high (e.g., by adjusting the melt temperature or the amount of water in the solvent), the drug containing nanoparticles are substantially uniformly suspended.

The therapeutic agent(s) within the release regions of the present invention need not be restricted to the location of the drug loaded nanoparticles. For example, in addition to being present within the drug loaded nanoparticles, therapeutic agent(s) can also be dissolved or dispersed within the polymeric carrier that surrounds the nanoparticles, as desired.

Moreover, in embodiments where a therapeutic agent (or agents) is provided within the polymeric carrier, this therapeutic agent (or agents) can be same as or different from the therapeutic agent (or agents) associated with the nanoparticles. Where they are the same, the therapeutic agent(s) within the polymeric carrier will generally be released first. For example, a therapeutic agent provided in the polymeric carrier may be used to create a burst of the therapeutic agent, followed by a release of the same therapeutic agent from the layered silicate nanoparticles at a slower rate, thereby achieving a sustained release profile.

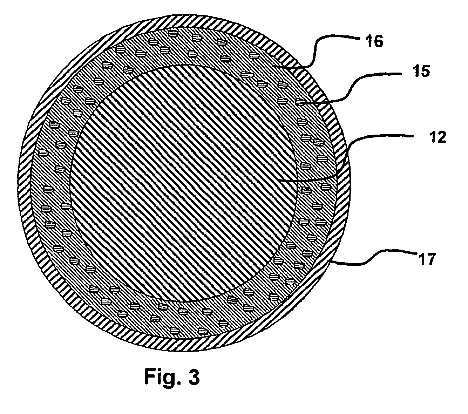

In accordance with some embodiments, the medical articles of the present invention are provided with a barrier layer that is disposed over the release layer. For example, Fig. 3 is a schematic cross-sectional view of a structural element 18, in accordance with an embodiment of the invention. As in Fig. 2 above, the release layer 16, which is directly adjacent the underlying structural member 12, includes a plurality of drug loaded nanoparticles 15 dispersed within a polymeric carrier. However, in Fig. 3, an additional polymeric barrier layer 17 is provided over the release layer 16, for example, to further the delay delivery of therapeutic agent from the medical article. Beneficially, the barrier layer comprises one or more polymers selected from the carrier layer polymers set forth above.

In addition to the agents discussed above, still other optional agents can be added to either the nanoparticles or to the polymeric carrier surrounding the same. Examples of such additional optional agents include radioisotopes for purposes of emitting radiation, as well as contrast agents, for instance, paramagnetic chelates such as gadolinium-DTPA complexes to make the composition MRI visible.

As indicated above, various techniques are available for forming the release regions of the present invention (and any optional barrier layer as well), including solution processing techniques and thermoplastic processing techniques.

Preferred solution processing techniques include solvent casting techniques, spin coating techniques, web coating techniques, solvent spraying techniques, dipping techniques, techniques involving coating via mechanical suspension, including air suspension coating (e.g., fluidized coating), transferring coating, inkjet/solenoid type coating techniques and electrostatic techniques. If desired, charge may be introduced to the nanoparticles as discussed above.

In many embodiments, a release-region-forming fluid, containing (a) solvent species, (b) polymer and (c) drug loaded nanoparticles, is applied to a medical article substrate, or to another template such as a mold or other release surface, and dried, thereby forming a release region. In other embodiments, for example, fiber forming techniques, a release region is formed without the aid of a substrate or other template.

Examples of solvent species for use in conjunction with the release-region-forming fluids include water and organic solvents such as hexane, heptane, toluene, dimethylsulfoxide, tetrahydrofuran, 1-methyl-2-pyrrolidone, cyclohexanone, ethanol, methanol, and chloroform, as well as combinations of the same.

If desired, the release-region-forming fluids can further comprise optional species, including additional therapeutic agents (which may be the same as or different from the therapeutic agents that are loaded onto and/or into the nanoparticles), contrast agents, radioisotopes, and so forth, as discussed above.

Where appropriate, techniques such as those listed above can be repeated or combined to build up a release regions to a desired thickness. The thickness of the release regions can be varied in other ways as well. For example, where the release region is formed by spraying, thickness can be increased by modification of coating process parameters, including increasing spray flow rate, slowing the movement between the substrate to be coated and the spray nozzle, providing repeated passes and so forth.

Where the release region is created using solution processing techniques, the solvent is removed after application, for example, by drying at room or elevated (e.g., 50°C) temperature, while under ambient pressure or under vacuum.

Thermoplastic processing techniques for forming the release regions of the present invention include molding techniques (for example, injection molding, rotational molding, and so forth), extrusion techniques (for example, extrusion, co-extrusion, multilayer extrusion, multi-lumen extrusion, and so forth) and casting.

Thermoplastic processing in accordance with the present invention typically comprises mixing or compounding, in one or more stages, the carrier polymer species, the drug loaded nanoparticles, and one or more of the following optional agents: additional therapeutic agents, contrast agents, radioisotopes, and so forth. The resulting mixture is then shaped into a medical article or a portion thereof. For example, a polymer melt may be formed by heating the carrier polymer species to a melt, which can then be mixed with the drug loaded nanoparticles as well as other optional agents. A common way of doing so is to apply mechanical shear using devices which are well known in the thermoplastic processing art, such as single screw extruders, twin screw extruders, banbury mixers, high-speed mixers, ross kettles, and so forth.

The carrier polymer and the nanoparticles are applied independently in some embodiments. For example, in some instances, a layer containing the carrier polymer species as well as any optional agents (e.g., additional therapeutic agents, contrast agents, radioisotopes, etc.) is applied to a substrate, for example, using one of the above-described thermoplastic or solvent-based techniques. Subsequently, nanoparticles are applied over the layer. Then, another layer containing the carrier polymer species (as well as any optional agents) is applied over the nanoparticles, thereby encapsulating the nanoparticles within the polymeric carrier and thus completing the formation of the release region.

In certain embodiments of the invention, the optional therapeutic agent is dissolved in a solvent and applied to a pre-existing release region, which can be formed using a variety of techniques including solution processing and thermoplastic processing techniques such as those discussed above, whereupon the optional therapeutic agent is imbibed into the release region.

"Therapeutic agents", "pharmaceutically active agents", "pharmaceutically active materials", "drugs" and other related terms may be used interchangeably herein and include genetic therapeutic agents, non-genetic therapeutic agents and cells. Therapeutic agents may be used singly or in combination. The therapeutic agent can be selected from suitable members of the lists of therapeutic agents to follow.

Exemplary non-genetic therapeutic agents for use in connection with the present invention include: (a) anti-thrombotic agents such as heparin, heparin derivatives, urokinase, and PPack (dextrophenylalanine proline arginine chloromethylketone); (b) anti-inflammatory agents such as dexamethasone, prednisolone, corticosterone, budesonide, estrogen, sulfasalazine and mesalamine; (c) antineoplastic/antiproliferative/anti-miotic agents such as paclitaxel, 5-fluorouracil, cisplatin, vinblastine, vincristine, epothilones, endostatin, angiostatin, angiopeptin, monoclonal antibodies capable of blocking smooth muscle cell proliferation, and thymidine kinase inhibitors; (d) anesthetic agents such as lidocaine, bupivacaine and ropivacaine; (e) anticoagulants such as D-Phe-Pro-Arg chloromethyl ketone, an RGD peptide-containing compound, heparin, hirudin, antithrombin compounds, platelet receptor antagonists, antithrombin antibodies, anti-platelet receptor antibodies, aspirin, prostaglandin inhibitors, platelet inhibitors and tick antiplatelet peptides; (f) vascular cell growth promoters such as growth factors, transcriptional activators, and translational promotors; (g) vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; (h) protein kinase and tyrosine kinase inhibitors (e.g., tyrphostins, genistein, quinoxalines); (i) prostacyclin analogs; (j) cholesterol-lowering agents; (k) angiopoietins; (l) antimicrobial agents such as triclosan, cephalosporins, aminoglycosides and nitrofurantoin; (m) cytotoxic agents, cytostatic agents and cell proliferation affectors; (n) vasodilating agents; (o) agents that interfere with endogenous vasoactive mechanisms; (p) inhibitors of leukocyte recruitment, such as monoclonal antibodies; (q) cytokines and (r) hormones.

Some exemplary non-genetic therapeutic agents include paclitaxel, sirolimus, everolimus, tacrolimus, cladribine, dexamethasone, estradiol, ABT-578 (Abbott Laboratories), trapidil, liprostin; Actinomcin D, Resten-NG, Ap-17, abciximab, clopidogrel and Ridogrel.

Exemplary genetic therapeutic agents for use in connection with the present invention include anti-sense DNA and RNA as well as DNA coding for: (a) anti-sense RNA, (b) tRNA or rRNA to replace defective or deficient endogenous molecules, (c) angiogenic factors including growth factors such as acidic and basic fibroblast growth factors, vascular endothelial growth factor, epidermal growth factor, transforming growth factor α and β, platelet-derived endothelial growth factor, platelet-derived growth factor, tumor necrosis factor α, hepatocyte growth factor and insulin-like growth factor, (d) cell cycle inhibitors including CD inhibitors, and (e) thymidine kinase ("TK") and other agents useful for interfering with cell proliferation. Also of interest is DNA encoding for the family of bone morphogenic proteins ("BMP's"), including BMP-2, BMP-3, BMP-4, BMP-5, BMP-6 (Vgr-1), BMP-7 (OP-1), BMP-8, BMP-9, BMP-10, BMP-11, BMP-12, BMP-13, BMP-14, BMP-15, and BMP-16. Currently preferred BMP's are any of BMP-2, BMP-3, BMP-4, BMP-5, BMP-6 and BMP-7. These dimeric proteins can be provided as homodimers, heterodimers, or combinations thereof, alone or together with other molecules. Alternatively, or in addition, molecules capable of inducing an upstream or downstream effect of a BMP can be provided. Such molecules include any of the "hedgehog" proteins, or the DNA's encoding them.

Vectors for delivery of genetic therapeutic agents include viral vectors such as adenoviruses, gutted adenoviruses, adeno-associated virus, retroviruses, alpha virus (Semliki Forest, Sindbis, etc.), lentiviruses, herpes simplex virus, replication competent viruses (e.g., ONYX-015) and hybrid vectors; and non-viral vectors such as artificial chromosomes and mini-chromosomes, plasmid DNA vectors (e.g., pCOR), cationic polymers (e.g., polyethyleneimine, polyethyleneimine (PEI)), graft copolymers (e.g., polyether-PEI and polyethylene oxide-PEI), neutral polymers PVP, SP1017 (SUPRATEK), lipids such as cationic lipids, liposomes, lipoplexes, nanoparticles, or microparticles, with and without targeting sequences such as the protein transduction domain (PTD).

Cells for use in connection with the present invention include cells of human origin (autologous or allogeneic), including whole bone marrow, bone marrow derived mono-nuclear cells, progenitor cells (e.g., endothelial progenitor cells), stem cells (e.g., mesenchymal, hematopoietic, neuronal), pluripotent stem cells, fibroblasts, myoblasts, satellite cells, pericytes, cardiomyocytes, skeletal myocytes or macrophage, or from an animal, bacterial or fungal source (xenogeneic), which can be genetically engineered, if desired, to deliver proteins of interest.

Numerous therapeutic agents, not necessarily exclusive of those listed above, have been identified as candidates for vascular treatment regimens, for example, as agents targeting restenosis. Such agents include one or more of the following: (a) Ca-channel blockers including benzothiazapines such as diltiazem and clentiazem, dihydropyridines such as nifedipine, amlodipine and nicardapine, and phenylalkylamines such as verapamil, (b) serotonin pathway modulators including: 5-HT antagonists such as ketanserin and naftidrofuryl, as well as 5-HT uptake inhibitors such as fluoxetine, (c) cyclic nucleotide pathway agents including phosphodiesterase inhibitors such as cilostazole and dipyridamole, adenylate/Guanylate cyclase stimulants such as forskolin, as well as adenosine analogs, (d) catecholamine modulators including α-antagonists such as prazosin and bunazosine, β-antagonists such as propranolol and α/β-antagonists such as labetalol and carvedilol, (e) endothelin receptor antagonists, (f) nitric oxide donors/releasing molecules including organic nitrates/nitrites such as nitroglycerin, isosorbide dinitrate and amyl nitrite, inorganic nitroso compounds such as sodium nitroprusside, sydnonimines such as molsidomine and linsidomine, nonoates such as diazenium diolates and NO adducts of alkanediamines, S-nitroso compounds including low molecular weight compounds (e.g., S-nitroso derivatives of captopril, glutathione and N-acetyl penicillamine) and high molecular weight compounds (e.g., S-nitroso derivatives of proteins, peptides, oligosaccharides, polysaccharides), as well as C-nitroso-compounds, O-nitroso-compounds, N-nitroso-compounds and L-arginine, (g) ACE inhibitors such as cilazapril, fosinopril and enalapril, (h) ATII-receptor antagonists such as saralasin and losartin, (i) platelet adhesion inhibitors such as albumin and polyethylene oxide, (j) platelet aggregation inhibitors including aspirin and thienopyridine (ticlopidine, clopidogrel) and GP IIb/IIIa inhibitors such as abciximab, epitifibatide and tirofiban, (k) coagulation pathway modulators including heparinoids such as heparin, low molecular weight heparin, dextran sulfate and β-cyclodextrin tetradecasulfate, thrombin inhibitors such as hirudin, hirulog, PPACK(D-phe-L-propyl-L-arg-chloromethylketone) and argatroban, FXa inhibitors such as antistatin and TAP (tick anticoagulant peptide), Vitamin K inhibitors such as warfarin, as well as activated protein C, (l) cyclooxygenase pathway inhibitors such as aspirin, ibuprofen, flurbiprofen, indomethacin and sulfinpyrazone, (m) natural and synthetic corticosteroids such as dexamethasone, prednisolone, methprednisolone and hydrocortisone, (n) lipoxygenase pathway inhibitors such as nordihydroguairetic acid and caffeic acid, (o) leukotriene receptor antagonists, (p) antagonists of E- and P-selectins, (q) inhibitors of VCAM-1 and ICAM-1 interactions, (r) prostaglandins and analogs thereof including prostaglandins such as PGE1 and PGI2 and prostacyclin analogs such as ciprostene, epoprostenol, carbacyclin, iloprost and beraprost, (s) macrophage activation preventers including bisphosphonates, (t) HMG-CoA reductase inhibitors such as lovastatin, pravastatin, fluvastatin, simvastatin and cerivastatin, (u) fish oils and omega-3-fatty acids, (v) free-radical scavengers/antioxidants such as probucol, vitamins C and E, ebselen, trans-retinoic acid and SOD mimics, (w) agents affecting various growth factors including FGF pathway agents such as bFGF antibodies and chimeric fusion proteins, PDGF receptor antagonists such as trapidil, IGF pathway agents including somatostatin analogs such as angiopeptin and ocreotide, TGF-β pathway agents such as polyanionic agents (heparin, fucoidin), decorin, and TGF-β antibodies, EGF pathway agents such as EGF antibodies, receptor antagonists and chimeric fusion proteins, TNF-α pathway agents such as thalidomide and analogs thereof, Thromboxane A2 (TXA2) pathway modulators such as sulotroban, vapiprost, dazoxiben and ridogrel, as well as protein tyrosine kinase inhibitors such as tyrphostin, genistein and quinoxaline derivatives, (x) MMP pathway inhibitors such as marimastat, ilomastat and metastat, (y) cell motility inhibitors such as cytochalasin B, (z) antiproliferative/antineoplastic agents including antimetabolites such as purine analogs (e.g., 6-mercaptopurine or cladribine, which is a chlorinated purine nucleoside analog), pyrimidine analogs (e.g., cytarabine and 5-fluorouracil) and methotrexate, nitrogen mustards, alkyl sulfonates, ethylenimines, antibiotics (e.g., daunorubicin, doxorubicin), nitrosoureas, cisplatin, agents affecting microtubule dynamics (e.g., vinblastine, vincristine, colchicine, paclitaxel and epothilone), caspase activators, proteasome inhibitors, angiogenesis inhibitors (e.g., endostatin, angiostatin and squalamine), rapamycin, cerivastatin, flavopiridol and suramin, (aa) matrix deposition/organization pathway inhibitors such as halofuginone or other quinazolinone derivatives and tranilast, (bb) endothelialization facilitators such as VEGF and RGD peptide, and (cc) blood rheology modulators such as pentoxifylline.

Numerous additional therapeutic agents are also disclosed in U.S. Patent No. 5,733,925 assigned to NeoRx Corporation.

In some embodiments of the invention, the medical article contains a hydrophobic therapeutic agent, a hydrophilic therapeutic agent, or both a hydrophobic therapeutic agent and a hydrophilic therapeutic agent. Specific examples of hydrophilic therapeutic agents include halofuginone·HBr and aqueous emulsions of therapeutic agents, among many others. Specific examples of hydrophobic therapeutic agents include paclitaxel and sirolimus, among many others. A wide range of therapeutic agent loadings can be used in connection with the release regions of the present invention, with the therapeutically effective amount being readily determined by those of ordinary skill in the art and ultimately depending, for example, upon the condition to be treated, the age, sex and condition of the patient, the nature of the therapeutic agent, the nature of the release regions, the nature of the medical article, and so forth.

Drug loading can be varied, for example, (a) by varying the concentration of the therapeutic agent within the layered silicate nanoparticles, (b) by varying the concentration of the layered silicate nanoparticles within the release region, and (c) by varying the concentration of therapeutic agent, if any, in the polymeric carrier.

In other aspects of the present invention, carbon nanotubes are used instead of, or in addition to, the layered silicate nanoparticles as described above.

### EXAMPLE

A drug loaded nanoparticle is provided by adding 25 mg halofuginone·HBr (a hydrophilic drug) to an aqueous polymer solution containing 25 mg hyaluronic acid (a hydrophilic polymer) in 200 mg water. 50 mg nanoclay, for example, montmorillonite clay from Nanocor, Arlington Heights, IL, patents Grade PGW, is then added into the solution and thoroughly mixed. The nanoclay is then dried, for example, by freeze-drying, and ground as needed to provide a fine nanoparticle powder. The nanoclay is not exfoliated into individual platelets prior to freeze-drying and grinding.

100 mg of SIBS (a hydrophobic polymer), which is made in accordance with the procedures described in U.S. Patent No. 5,741,331, U.S. Patent No. 4,946,899 and U.S. Patent No. 6,545,097, is dissolved in 150 µl of toluene to obtain a uniform clear SIBS solution. 80 mg of drug loaded nanoparticles from the prior paragraph is added into the SIBS solution. The nanoparticles suspended in the SIBS solution are stable and ready for coating.

A stent is coated using a fluidized bed process like that described in U.S. Patent Appln. No. 2001/0022988 entitled "Device and method for protecting medical devices during a coating process". Various thickness of coating can be obtained ranging from 5 to 50 µm.

## Claims

1. A medical article comprising a release region, said release region comprising (a) polymeric carrier comprising a first polymer and (b) drug loaded nanoparticles dispersed within said polymeric carrier, said drug loaded nanoparticles comprising layered silicate particles and a first therapeutic agent, associated with the silicate particles in a way such that it occupies the spaces between adjacent layers of the silicate particles.

2. The medical article of claim 1, wherein the unmodified layered silicate particles are more hydrophilic than the therapeutic agent, and wherein the layered silicate material is modified as to render it more hydrophobic by exchange of endogenous inorganic cations with one or more molecule species comprising a positive charge and a hydrophobic domain.

3. The medical article of claim 1,
wherein said first therapeutic agent is a hydrophilic therapeutic agent and said first polymer is a hydrophobic polymer.

4. The medical article of claim 3,
wherein said medical article is a vascular medical device, wherein said first therapeutic agent is haIofuginone-HBr, and wherein said first polymer is a polyolefin-polyvinylaromatic block copolymer.

5. The medical article of claim 1,
wherein said first therapeutic agent is a hydrophobic therapeutic agent and said first polymer is a hydrophilic polymer, or
wherein said polymeric carrier further comprises said first therapeutic agent, or
wherein said release region is disposed over at least a portion of a medical article substrate, or
wherein said layered silicate material comprises synthetic or naturally occurring smectite, or
wherein said layered silicate material comprises a natural or synthetic silicate material selected from bentonite, aliettite, vermiculite, swinefordite, montmorillonite, yakhontovite, nontronite, beidellite, volkonskoite, stevensite, hectorite, saponite, laponite, sauconite, magadiite, kenyaite and ledikite.

6. The medical article of claim 1, further comprising a second polymer.

7. The medical article of claim 6, wherein said polymeric carrier further comprises said second polymer.

8. The medical article of claim 6, wherein said nanoparticles further comprise said second polymer, and wherein said second polymer is hydrophobic and said first polymer is hydrophilic, or said second polymer is hydrophilic and said first polymer is hydrophobic, wherein said medical article may be a vascular medical device, wherein said first therapeutic agent is halofuginone-HBr, wherein said first polymer is a polyolefin-polyvinylaromatic block copolymer, and wherein said second polymer is a hydrophilic polymer selected from hyaluronic acid, collagen, heparin, chondroitin sulfate, phosphoro choline, dextran, and polyethylene oxide.

9. The medical article of claim 1, further comprising a second therapeutic agent.

10. The medical article of claim 9,
wherein said polymeric carrier further comprises said second therapeutic agent, wherein said first therapeutic agent preferably is hydrophilic and said second therapeutic agent preferably is hydrophobic, or
wherein said nanoparticles further comprise said second therapeutic agent, wherein said first and second therapeutic agents preferably are hydrophilic.

11. The medical article of claim 1, wherein said medical article is an implantable or insertable medical device.

12. The medical article of claim 11, wherein said implantable or insertable medical device is adapted for implantation or insertion into the coronary or peripheral vasculature.

13. The medical article of claim 12, wherein
said implantable or insertable medical device is adapted for implantation or insertion into the esophagus, trachea, colon, biliary tract, urinary tract, prostate or brain, or
said implantable or insertable medical device is selected from a catheter, a guide wire, a balloon, a filter, a stent, a stent graft, a vascular graft, a vascular patch, a shunt, an electrode, a heart valve, a circulation pump, and an intraluminal paving system, or
said therapeutic agent is selected from an anti-thrombotic agent, an anti-proliferative agent, an anti-inflammatory agent, an anti-migratory agent, an agent affecting extracellular matrix production and organization, an antineoplastic agent, an anti-mitotic agent, an anesthetic agent, an anti-coagulant, a vascular cell growth promoter, a vascular cell growth inhibitor, a cholesterol-lowering agent, a vasodilating agent, and an agent that interferes with endogenous vasoactive mechanisms.

14. Use of an article according to claim 1 for the production of a medical device for the treatment of a mammalian tissue or organ selected from tumors, heart, coronary and peripheral vascular system, lungs, trachea, esophagus, brain, liver, kidney, bladder, urethra and ureters, eye, intestines, stomach, pancreas, ovary, prostate, skeletal muscle, smooth muscle, breast, dermal tissue, cartilage, and bone, in particular for the treatment of tumors or of restenosis.

15. A method of providing the medical article of claim 1 comprising:
providing a release-region-forming fluid comprising (a) said first polymer species and (b) said drug loaded nanoparticles; and
applying said release-region-forming fluid to a medical article substrate or to a releasable template.

## Patentansprüche

1. Ein medizinischer Gegenstand, umfassend eine Abgaberegion, wobei die Abgaberegion (a) polymeren Träger, umfassend ein erstes Polymer, und (b) medikamentenbeladene Nanoteilchen, dispergiert im polymeren Träger, umfasst, wobei die medikamentenbeladenen Nanoteilchen Schichtsilikat-Teilchen und ein erstes therapeutisches Mittel umfassen, das so mit den Silikatteilchen assoziiert ist, dass es die Räume zwischen benachbarten Schichten der Silikatteilchen einnimmt.

2. Der medizinische Gegenstand nach Anspruch 1, wobei die unmodifizierten Schichtsilikat-Teilchen hydrophiler sind als das therapeutische Mittel und wobei das Schichtsilikat-Material so modifiziert ist, dass es durch den Austausch endogener anorganischer Kationen, durch einen oder mehrere Molekültypen, umfassend eine positive Ladung und einen hydrophoben Bereich, hydrophober gemacht wird.

3. Der medizinische Gegenstand nach Anspruch 1,
wobei das erste therapeutische Mittel ein hydrophiles therapeutisches Mittel ist und das erste Polymer ein hydrophobes Polymer ist.

4. Der medizinische Gegenstand nach Anspruch 3,
wobei der medizinische Gegenstand eine vaskuläre medizinische Vorrichtung ist, wobei das erste therapeutische Mittel Halofuginon-HBr ist und wobei das erste Polymer ein Polyolefin-polyvinylaromatisches Blockcopolymer ist.

5. Der medizinische Gegenstand nach Anspruch 1,
wobei das erste therapeutische Mittel ein hydrophobes therapeutisches Mittel ist und das erste Polymer ein hydrophiles Polymer ist, oder
wobei der polymere Träger ferner das erste therapeutische Mittel umfasst oder
wobei die Abgaberegion über mindestens einem Bereich eines medizinischen Gegenstand-Substrats angeordnet ist oder
wobei das Schichtsilikat-Material synthetischen oder natürlich vorkommenden Smektit umfasst oder
wobei das Schichtsilikat-Material ein natürliches oder synthetisches Silikatmaterial umfasst, ausgewählt aus Bentonit, Aliettit, Vermiculit, Swinefordit, Montmorillonit, Yakhontovit, Nontronit, Beidellit, Volkonskoit, Stevensit, Hectorit, Saponit, Laponit, Sauconit, Magadiit, Kenyait und Ledikit.

6. Der medizinische Gegenstand nach Anspruch 1, ferner umfassend ein zweites Polymer.

7. Der medizinische Gegenstand nach Anspruch 6, wobei der polymere Träger ferner das zweite Polymer umfasst.

8. Der medizinische Gegenstand nach Anspruch 6, wobei die Nanoteilchen ferner das zweite Polymer umfassen und wobei das zweite Polymer hydrophob ist und das erste Polymer hydrophil ist oder das zweite Polymer hydrophil ist und das erste Polymer hydrophob ist, wobei der medizinische Gegenstand eine vaskuläre medizinische Vorrichtung sein kann, wobei das erste therapeutische Mittel Halofuginon-HBr ist, wobei das erste Polymer ein Polyolefin-polyvinylaromatisches Blockcopolymer ist und wobei das zweite Polymer ein hydrophiles Polymer ist, ausgewählt aus Hyaluronsäure, Collagen, Heparin, Chondroitinsulfat, Phosphorcholin, Dextran und Polyethylenoxid.

9. Der medizinische Gegenstand nach Anspruch 1, ferner umfassend ein zweites therapeutisches Mittel.

10. Der medizinische Gegenstand nach Anspruch 9,
wobei der polymere Träger ferner das zweite therapeutische Mittel umfasst,
wobei das erste therapeutische Mittel vorzugsweise hydrophil ist und das zweite therapeutische Mittel vorzugsweise hydrophob ist, oder
wobei die Nanoteilchen ferner das zweite therapeutische Mittel umfassen, wobei die ersten und zweiten therapeutischen Mittel vorzugsweise hydrophil sind.

11. Der medizinische Gegenstand nach Anspruch 1, wobei der medizinische Gegenstand eine implantierbare oder einführbare medizinische Vorrichtung ist.

12. Der medizinische Gegenstand nach Anspruch 11, wobei die implantierbare oder einführbare medizinische Vorrichtung zum Implantieren oder Einführen in das koronare oder periphere Gefäßsystem angepasst ist.

13. Der medizinische Gegenstand nach Anspruch 12, wobei
die implantierbare oder einführbare medizinische Vorrichtung zum Implantieren oder Einführen in Speiseröhre, Luftröhre, Dickdarm, Gallengang, Harnwege, Prostata oder Gehirn angepasst ist oder
die implantierbare oder einführbare medizinische Vorrichtung ausgewählt ist aus einem Katheter, einem Führungsdraht, einem Ballon, einem Filter, einem Stent, einem Stenttransplantat, einem Gefäßtransplantat, einem Gefäßpatch, einem Shunt, einer Elektrode, einer Herzklappe, einer Zirkulationspumpe, und einem intraluminalen Befestigungssystem oder
das therapeutische Mittel ausgewählt ist aus einem Antithrombosemittel, einem proliferationshemmenden Mittel, einem entzündungshemmenden Mittel, einem migrationshemmenden Mittel, einem Mittel, das extrazelluläre Matrixproduktion und -organisation beeinflusst, einem antineoplastischen Mittel, einem mitosehemmenden Mittel, einem anästhetischen Mittel, einem Gerinnungshemmer, einem Gefäßzellenwachstumsförderer, einem Gefäßzellenwachstumsinhibitor, einem Cholesterin senkenden Mittel, einem gefäßerweiternden Mittel und einem Mittel, das mit endogenen gefäßaktiven Mechanismen interferiert.

14. Verwendung eines Gegenstands gemäß Anspruch 1 zur Herstellung einer medizinischen Vorrichtung zur Behandlung eines Säugergewebes oder Organs, ausgewählt aus Tumoren, Herz, koronarem und peripherem Gefäßsystem, Lungen, Luftröhre, Speiseröhre, Gehirn, Leber, Niere, Blase, Harnröhre und Harnleitern, Auge, Gedärmen, Magen, Bauchspeicheldrüse, Eierstock, Prostata, Skelettmuskel, glattem Muskel, Brust, Hautgewebe, Knorpel und Knochen, insbesondere zur Behandlung von Tumoren oder Restenose.

15. Ein Verfahren zum Bereitstellen des medizinischen Gegenstands nach Anspruch 1, umfassend:
Bereitstellen einer Abgaberegion bildenden Flüssigkeit, umfassend (a) den ersten Polymertyp und (b) die medikamentenbeladenen Nanoteilchen; und
Auftragen der Abgaberegion bildenden Flüssigkeit auf ein medizinischer Gegenstand-Substrat oder auf eine abtrennbare Matrize.

## Revendications

1. Article médical comprenant une région de libération, ladite région de libération comprenant (a) un porteur polymère comprenant un premier polymère et (b) des nanoparticules chargées de médicament dispersées à l'intérieur dudit porteur polymère, lesdites nanoparticules chargées de médicament comprenant des particules de silicate en couche et un premier agent thérapeutique, associé avec les particules de silicate de sorte qu'il occupe des espaces entre les couches adjacentes des particules de silicate.

2. Article médical selon la revendication 1, dans lequel les particules de silicate en couche non modifiées sont plus hydrophiles que l'agent thérapeutique, et dans lequel le matériau de silicate en couche est modifié afin de le rendre plus hydrophobe par échange de cations inorganiques endogènes avec une ou plusieurs espèces moléculaires comprenant une charge positive et un domaine hydrophobe.

3. Article médical selon la revendication 1, dans lequel ledit premier agent thérapeutique est un agent thérapeutique hydrophile et ledit premier polymère est un polymère hydrophobe.

4. Article médical selon la revendication 3, dans lequel ledit article médical est un dispositif médical vasculaire, dans lequel ledit premier agent thérapeutique est l'halofuginone-HBr, et dans lequel ledit premier polymère est un copolymère bloc de polyoléfine-polyvinylaromatique.

5. Dispositif médical selon la revendication 1,
dans lequel ledit premier agent thérapeutique est un agent thérapeutique hydrophobe et ledit premier polymère est un polymère hydrophile, ou bien
dans lequel ledit porteur polymère comprend en outre ledit premier agent thérapeutique, ou bien
dans lequel ladite région de libération est disposée sur au moins une partie d'un substrat d'article médical, ou bien
dans lequel ledit matériau de silicate en couche comprend la smectite synthétique ou naturelle, ou bien
dans lequel ledit matériau de silicate en couche comprend un matériau de silicate naturel ou synthétique choisi parmi la bentonite, l'aliettite, la vermiculite, la swinefordite, la montmorillonite, la yakhontovite, la nontronite, la beidellite, la volkonskoite, la stévensite, l'hectorite, la saponite, la laponite, la sauconite, la magadiite, la kenyaite et la ledikite.

6. Article médical selon la revendication 1, comprenant en outre un deuxième polymère.

7. Article médical selon la revendication 6, dans lequel ledit porteur polymère comprend en outre ledit deuxième polymère.

8. Article médical selon la revendication 6, dans lequel lesdites nanoparticules comprennent en outre ledit deuxième polymère, et dans lequel ledit deuxième polymère est hydrophobe et ledit premier polymère est hydrophile, ou bien ledit deuxième polymère est hydrophile et ledit premier polymère est hydrophobe, dans lequel ledit article médical peut être un dispositif médical vasculaire, dans lequel ledit premier agent thérapeutique est l'halofuginone-HBr, dans lequel ledit premier polymère est un copolymère bloc de polyoléfine-polyvinylaromatique, et dans lequel ledit deuxième polymère est un polymère hydrophile choisi parmi l'acide hyaluronique, le collagène, l'héparine, le sulfate de chondroïtine, la phosphorylcholine, le dextran et l'oxyde de polyéthylène.

9. Article médical selon la revendication 1, comprenant en outre un deuxième agent thérapeutique.

10. Article médical selon la revendication 9,
dans lequel ledit porteur polymère comprend en outre ledit, deuxième agent thérapeutique, dans lequel ledit premier agent thérapeutique est de préférence hydrophile et ledit deuxième agent thérapeutique est de préférence hydrophobe, ou bien
dans lequel lesdites nanoparticules comprennent en outre ledit deuxième agent thérapeutique, dans lequel lesdits premier et deuxième agents thérapeutiques sont de préférence hydrophiles.

11. Article médical selon la revendication 1, dans lequel ledit article médical est un dispositif médical implantable ou insérable.

12. Article médical selon la revendication 11, dans lequel ledit dispositif médical implantable ou insérable est adapté pour l'implantation ou l'insertion dans le système vasculaire coronaire ou périphérique.

13. Article médical selon la revendication 12, dans lequel :
ledit dispositif médical implantable ou insérable est adapté pour l'implantation ou l'insertion dans l'oesophage, la trachée, le côlon, les voies biliaires, les voies urinaires, la prostate ou le cerveau, ou bien
ledit dispositif médical implantable ou insérable est choisi parmi un cathéter, un fil-guide, un ballonnet, un filtre, un stent, un greffon endoprothétique, une greffe vasculaire, un timbre vasculaire, une dérivation, une électrode, une valvule cardiaque, une pompe de circulation et un système préparatoire intraluminal, ou bien
ledit agent thérapeutique est choisi parmi un agent anti-thrombogène, un agent anti-prolifératif, un agent anti-inflammatoire, un agent anti-migratoire, un agent affectant la production et l'organisation de la matrice extracellulaire, un agent anti-néoplasique, un agent anti-mitotique, un agent anesthésique, un anti-coagulant, un promoteur de la croissance cellulaire vasculaire, un inhibiteur de croissance cellulaire vasculaire, un hypocholestérolémiant, un vasodilatateur, et un agent qui interfère avec les mécanismes vasoactifs endogènes.

14. Utilisation d'un article selon la revendication 1 pour la production d'un dispositif médical pour le traitement d'un tissu ou organe de mammifère choisi parmi les tumeurs, le coeur, le système vasculaire coronaire et périphérique, les poumons, la trachée, l'oesophage, le cerveau, le foie, le rein, la vessie, l'urètre et les uretères, l'oeil, les intestins, l'estomac, le pancréas, l'ovaire, la prostate, les muscles squelettiques, le muscle lisse, le sein, le tissu cutané, le cartilage et l'os, en particulier pour le traitement des tumeurs ou de resténose.

15. Procédé pour proposer l'article médical selon la revendication 1, comprenant les étapes consistant à :
prévoir un fluide de formation de région de libération comprenant (a) ladite première espèce de polymère et (b) lesdites nanoparticules chargées en médicament : et
appliquer ledit fluide de formation de région de libération sur un substrat d'article médical ou sur un gabarit libérable.
